(19) Europäisches Patentamt
European Patent Office
Office européen des brevets

(11) Veröffentlichungsnummer : **0 026 908**
**B1**

# (12) EUROPÄISCHE PATENTSCHRIFT

(45) Veröffentlichungstag der Patentschrift : **22.06.83**

(51) Int. Cl.³ : **C 07 D233/58**, C 07 D233/60, C 07 D233/61

(21) Anmeldenummer : **80105908.0**

(22) Anmeldetag : **30.09.80**

## (54) Verfahren zur Herstellung von in 1-Stellung substituierten Imidazolen.

(30) Priorität : **08.10.79 DE 2940709**

(43) Veröffentlichungstag der Anmeldung : **15.04.81 Patentblatt 81/15**

(45) Bekanntmachung des Hinweises auf die Patenterteilung : **22.06.83 Patentblatt 83/25**

(84) Benannte Vertragsstaaten : **BE CH DE FR GB IT LI NL**

(56) Entgegenhaltungen :
**ARCHIV DER PHARMAZIE, Band 309, Nr. 5, 1976 W. STOECK et al. « Imidazolsynthesen, 8. Mitt. N-Substituierte Imidazole nach Weidenhagen » Seiten 421 bis 426**
**ARCHIV DER PHARMAZIE, Band 307, Nr. 12 1974 V. STOECK et al. « N-Substituierte Imidazole aus Aldehyden, 1,2-Diketonen, primären Aminen und flüssigem Ammoniak » Seiten 922 bis 925**
**JOURNAL AND PROCEEDINGS OF THE ROYAL SOCIETY OF NEW SOUTH WALES, vol. LXXIV, 1941, Sydney F. LIONS et al. « A Direct Synthesis of 1 : 2 : 4 : 5-Tetrasubstituted Iminazoles » Seiten 365 bis 372**

(73) Patentinhaber : **BASF Aktiengesellschaft**
**Carl-Bosch-Strasse 38**
**D-6700 Ludwigshafen (DE)**

(72) Erfinder : **Graf, Fritz, Dr.**
**Im Rothschild 33**
**D-6720 Speyer (DE)**
Erfinder : **Hupfer, Leopold, Dr.**
**Waltershoehe 3**
**D-6701 Friedelsheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 026 908 B1

# Verfahren zur Herstellung von in 1-Stellung substituierten Imidazolen

Diese Erfindung betrifft ein neues Verfahren zur Herstellung von in 1-Stellung substituierten Imidazolen.

In 1-Stellung substituierte Imidazole, wie N-Alkylimidazole, kann man durch katalytische Alkylierung von Imidazolen mit Alkanolen bei höherer Temperatur und Druck oder durch Umsetzung von Imidazolen mit Alkylchloriden herstellen. Da diese Verfahren als Vorstufe das am Stickstoff nicht substituierte Imidazol benötigen, war nach einem Verfahren zu suchen, das es erlaubt, in einer Reaktionsstufe unmittelbar die N-substituierten Imidazole herzustellen.

Man hat zwar schon versucht, auch in 1-Stellung substituierte Imidazole aus den Ausgangsstoffen für die Imidazolsynthese, wie Diacetyl, Ammoniak, Aldehyd und Alkylamin herzustellen, wobei man zuerst aus Diacetyl und dem Amin die Schiffsche Base gebildet hat, die dann mit dem Umsetzungsprodukt aus dem Aldehyd und Ammoniak umgesetzt wurde. Bei diesem in J. Proc. Royal Soc. N.S.W. 74 (1941) Seiten 365 bis 372 beschriebenen Verfahren werden die in 1-Stellung substituierten Imidazolen jedoch nur in geringen Ausbeuten erhalten.

Ungünstige Ergebnisse werden auch erhalten, wenn man in 1-Stellung substituierte Imidazole nach dem in Archiv der Pharmazie, Band 309 (1976) Seiten 421 bis 425 beschriebenen Verfahren durch Umsetzung von α-Hydroxy-, α-Acyloxy- oder α-Halogenketonen mit Ammoniak, Aldehyden und primären Aminen in Gegenwart von erheblichen Mengen an Kupfer-(II)-acetat herstellt. Bei diesem Verfahren werden die in 1-Stellung substituierten Imidazole in nur 10 bis 25 %iger Ausbeute gebildet.

Es wurde nun gefunden, daß man in 1-Stellung substituierte Imidazole vorteilhafter, insbesondere in erheblich besserer Ausbeute herstellen kann, wenn man eine α-Dicarbonylverbindung, Ammoniak, einen Aldehyd und ein primäres Amin in wäßrigem Medium in einer Stufe bei Temperaturen von 20 bis 150 °C umsetzt.

Daß man die in 1-Stellung substituierten Imidazole nach dem erfindungsgemäßen Verfahren in guter Ausbeute erhält, mußte überraschen, da die geringen Ausbeuten bei der oben erwähnten Synthese aus α-Hydroxy-, α-Acyloxy- oder α-Halogenketonen weder durch eine Änderung der Konzentrationen der Reaktionspartner noch durch eine Variation der Reaktionsbedingungen erhöht werden konnten (s. Arch. Pharm. Bd. 309 (1976) Seite 423, Zeilen 6 bis 8).

Nach dem neuen Verfahren lassen sich in 1-Stellung substituierte Imidazole herstellen, die in den Stellungen 2, 4 und 5 substituiert sein können. Das sind z. B. Verbindungen der Formel

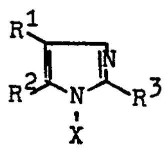

in der $R^1$, $R^2$ und $R^3$ Wasserstoffatome oder aliphatische oder aromatische Reste und X einen aliphatischen, cycloaliphatischen oder aromatischen Rest bedeuten.

Als Ausgangsstoffe kommen z. B. α-Dicarbonylverbindungen der Formel $R^1$—CO—CO—$R^2$ in Betracht, in der die Reste $R^1$ und $R^2$ die obengenannte Bedeutung haben. Aliphatische Reste sind z. B. gegebenenfalls substituierte geradkettige oder verzweigte Alkylreste mit 1 bis 5 C-Atomen. Aromatische Reste sind gegebenenfalls substituierte aromatische Reste, wie Phenyl- oder Naphthylreste. Als Substituenten der aliphatischen und aromatischen Reste kommen z. B. HO-, $H_3COOC$- und $H_3CO$-Gruppen in Betracht. Geeignete α-Dicarbonylverbindungen sind z. B. Glyoxal, Diacetyl, Benzil oder Methylglyoxal.

Als Aldehyde sind z. B. Verbindungen der Formel $R^3$—CHO geeignet, in der $R^3$ die obengenannte Bedeutung hat. Als aliphatische Reste können die Aldehyde z. B. geradkettige oder verzweigte Alkylreste enthalten, wie solche mit 1 bis 8 C-Atomen, die z. B. durch Phenyl-, HO-, $H_3COOC$- oder $H_3CO$-Gruppen substituiert sein können. Als aromatischen Rest können die Aldehyde z. B. einen Phenylrest enthalten, der durch Alkyl-, HO- oder $H_3COOC$-Gruppen substituiert sein kann. Geeignete Aldehyde sind z. B. Formaldehyd, Acetaldehyd, Propionaldehyd, Isobutyraldehyd und Benzaldehyd.

Die als Ausgangskomponente zu verwendenden primären Aminen sind z. B. Monoamine der Formel X—$NH^2$ der aliphatischen, cycloaliphatischen oder aromatischen Reihe. Als aliphatischer Rest kommt ein geradkettiger oder verzweigter gesättigter oder ungesättigter Kohlenwasserstoffrest mit 1 bis 20 C-Atomen in Betracht, der z. B. durch HO-, $(CH_3)NH$-, $(CH_3)_2N$-, $H_5C_2NH$-, $H_5C_2O$-, $H_3CO$- oder Äthergruppen substituiert sein kann. Cycloaliphatische Reste sind z. B. Cyclohexyl und Norbornyl. Aromatische Reste sind z. B. Phenylreste, die durch $H_3CO$-, $(CH_3)_2N$-, $H_5C_2NH$- oder $H_3COOC$-Gruppen substituiert sein können. Geeignete Amine der genannten Art sind z. B. Methylamin, Äthylamin, Propylamin, Octylamin, Dodecylamin, Anilin, Methoxyäthylamin, Äthoxyäthylamin, Dimethylaminopropylamin, 3-Amino-3-methyl-1-butin, 2-Hydroxy-2'-aminodiäthyläther, 3-Aminopropanol, 3,3-Dimethoxy-2-propylamin, 3-Methylamino-1-propylamin, Anthranilsäuremethylester und Cyclohexylamin. Ammoniak kommt zweckmäßig in wäßriger Lösung zur Anwendung.

Die genannten vier Ausgangsstoffe werden zweckmäßig in stöchiometrischen Mengen, d. h. im Molverhältnis 1 : 1 : 1 : 1 eingesetzt. Abweichungen von diesen Mengenverhältnissen sind möglich, bringen aber keinen Vorteil.

Die Umsetzung wird in wäßrigem Medium bei Temperaturen von 20 bis 150 °C, vorzugsweise von 70 bis 120 °C vorgenommen. Dabei kann es zweckmäßig sein, als Lösungsvermittler unter den Umsetzungsbedingungen mit den Ausgangsstoffen nicht reagierende Lösungsmittel, wie mit Wasser mischbare Alkohole zuzusetzen. Geeignete Lösungsvermittler sind z. B. Äthanol, Methanol und Propanol.

Nach der Umsetzung, die in der Regel nach etwa 30 Minuten beendet ist, arbeitet man das Reaktionsgemisch zur Isolierung der Imidazole z. B. durch Destillation oder Extraktion auf.

Nach dem neuen Verfahren lassen sich die in 1-Stellung substituierten Imidazole glatt und in guter Ausbeute herstellen. Überraschenderweise werden dabei nur geringe Mengen der in 1-Stellung nicht substituieren Imidazole gebildet. Die Verfahrensprodukte sind wertvolle Zwischenprodukte, z. B. für die Herstellung von Heilmitteln oder Pflanzenschutzmitteln.

## Beispiel 1

Herstellung von 1-Methylimidazol

In einem Rührkolben werden bei 70 °C die Mischungen aus 145 Teilen 40 %iger wäßriger Glyoxal-Lösung und 75 Teilen 40 %iger wäßriger Formaldehydlösung sowie 382,5 Teilen Ammoniakwasser 4,4 %ig und 77,5 Teilen 40 %iger wäßriger Methylamin-Lösung während 30 Minuten gemischt. Nach beendeter Zugabe wird noch weitere 30 Minuten bei 70 °C nachgerührt. Das Reaktionsgemisch wird anschließend destillativ aufgearbeitet. Man erhält 67,8 Teile N-Methylimidazol (Kp. 63°-65 °C ; 3 Torr) in einer Reinheit von 98,5 % entsprechend einer Ausbeute von 81,4 %.

## Beispiel 2

Herstellung von 1-Phenylimidazol

Eine Mischung aus 93,1 Teilen Anilin und 68 Teilen Ammoniakwasser 25 %ig, gelöst in 100 Teilen Propanol wird gleichzeitig mit einer Mischung aus 145 Teilen 40 %iger wäßriger Glyoxyl-Lösung und 75 Teilen 40 %iger wäßriger Formaldehyd-Lösung bei 80 °C während 30 Minuten in 200 Teile Propanol eingerührt. Anschließend hält man noch 30 Minuten bei 80 °C. Das Lösungsmittel wird im Vakuum abgezogen und der Rückstand fraktioniert destilliert. Es werden 100,9 Teile N-Phenylimidazol (Kp. 128 °C, 2 Torr) erhalten, entsprechend einer Ausbeute von 70,0 %.

## Beispiel 3

Herstellung von 1-Octylimidazol

64,6 Teile n-Octylamin und 34 Teile Ammoniakwasser 25 %ig werden in 186 Teilen Propanol gelöst und gleichzeitig mit einer Mischung aus 72,5 Teilen 40 %iger wäßriger Glyoxal-Lösung und 37,5 Teilen 40 %iger wäßriger Formaldehyd-Lösung während 30 Minuten in einen Rührkolben eingetropft. Die Temperatur hält man bei 65 °C. Man rührt 20 Minuten bei 65 °C nach und arbeitet anschließend destillativ auf. Es werden 64,2 Teile 1-Octylimidazol (Kp. 110 °C, 1 Torr) erhalten, entsprechend einer Ausbeute von 71,3 % der Theorie.

## Beispiel 4

Herstellung von 1-Methylimidazol

In einen Autoklaven werden bei 110 °C während 30 Minuten gleichzeitig die Mischungen aus 145 Teilen 40 %iger wäßriger Glyoxal-Lösung und 75 Teilen 40 %iger wäßriger Formaldehyd-Lösung sowie 77,5 Teilen 40 %iger wäßriger Methylamin-Lösung und 382,5 Teilen Ammoniakwasser 4,4 %ig in 100 Teile vorgelegtes Wasser eingeleitet. Nach weiteren 30 Minuten bei 110 °C wird destillativ aufgearbeitet. Man erhält 76,8 Teile 1-Methylimidazol, entsprechend 93,7 % der Theorie.

## Beispiel 5

Herstellung von 1,4,5-Trimethylimidazol

88 Teile Diacetyl und 75 Teile 40 %iger wäßriger Formaldehyd-Lösung werden in 50 Teilen Propanol gelöst und während 30 Minuten gleichzeitig mit einer Mischung aus 77,5 Teilen 40 %iger wäßriger Methylamin-Lösung und 170 Teilen Ammoniakwasser 10 %ig bei 70 °C in 100 Teile Propanol eingerührt.

Nach weiteren 30 Minuten bei 70 °C wird destillativ aufgearbeitet. Man erhält 63,6 Teile 1,4,5-Trimethylimidazol (Kp. 73 °C, 3 Torr) entsprechend einer Ausbeute von 57,8 % der Theorie.

Beispiel 6

Herstellung von 1-Dodecylimidazol

92,5 Teile Dodecylamin und 34 Teile Ammoniakwasser 25 %ig werden in 100 Teilen Propanol gelöst und bei 80 °C gleichzeitig mit einer Mischung von 72,5 Teilen 40 %iger wäßriger Glyoxal-Lösung und 37,5 Teilen 40 %iger wäßriger Formaldehyd-Lösung zu 200 Teilen Propanol während 30 Minuten zugetropft. Das Gemisch wird 20 Minuten nachgerührt. Nach destillativer Aufarbeitung werden 76,5 Teile N-Dodecylimidazol (Kp. 152 °C, 2 Torr) erhalten, entsprechend 64,8 % der Theorie.

Beispiel 7

Herstellung von 1,2,4,5-Tetramethylimidazol

Eine Mischung aus 86 Teilen Diacetyl und 44 Teilen Acetaldehyd wird gleichzeitig mit einer Mischung aus 77,5 Teilen 40 %iger wäßriger Methylamin-Lösung und 68 Teilen 25 %igem Ammoniakwasser während 30 Minuten bei 50 °C in 300 Teile Propanol eingetropft. Nach weiteren 30 Minuten bei 50 °C wird das Gemisch destillativ aufgearbeitet. Es werden 52 g 1,2,4,5-Tetramethylimidazol erhalten (Kp. 110°/1,5 Torr) entsprechend 45,4 % der Theorie.

Beispiel 8

Herstellung von 1-(3,3-Dimethyl-1-propin-1-yl)-imidazol

Ein Gemisch aus 83 Teilen 3-Amino-3-methyl-1-butin und 170 Teilen 10 %igem Ammoniakwasser wird gleichzeitig mit einem Gemisch aus 145 Teilen 40 %iger wäßriger Glyoxal-Lösung und 75 Teilen 40 %iger wäßriger Formaldehyd-Lösung während 20 Minuten unter Rühren bei 50 °C in 100 Teile vorgelegtes Wasser eingetropft. Nach beendeter Zugabe wird noch 30 Minuten bei 50 °C nachgerührt ; anschließend wird destillativ aufgearbeitet. Man erhält 77,2 g 1-(3,3-Dimethyl-1-propin-1-yl)-imidazol (Kp 118°/15 Torr). Das entspricht einer Ausbeute von 66,0 % der Theorie.

Beispiel 9

Herstellung von 1-(2'-Hydroxyäthoxy-1-äthyl)-imidazol

Ein Gemisch aus 105 Teilen 2-Hydroxy-2'-amino-diäthyläther und 170 Teilen Ammoniakwasser 10 %ig wird gleichzeitig mit einem Gemisch aus 145 Teile 40 %iger wäßriger Glyoxal-Lösung und 75 Teilen 40 %iger wäßriger Formaldehyd-Lösung bei 70 °C in 100 Teile vorgelegtes Wasser eingerührt. Anschließend wird noch 30 Minuten bei 70 °C nachgerührt. Die destillative Aufarbeitung ergibt 131 Teile 1-(2'-Hydroxy äthoxy-1-äthyl)imidazol (Kp 165°/1 Torr) entsprechend einer Ausbeute von 84,0 %.

Beispiel 10

Herstellung von 1-(3-Hydroxy-1-propyl)-imidazol

Eine Mischung aus 75 Teilen 3-Amino-1-propanol und 170 Teilen Ammoniakwasser 10 %ig wird während 30 Minuten gleichzeitig mit einer Mischung aus 145 Teilen 40 %iger wässriger Glyoxal-Lösung und 75 Teilen 40 %iger wäßriger Formaldehyd-Lösung bei 70 °C unter Rühren in 100 Teilen Wasser eingetropft. Nachdem noch 30 Minuten bei 70 °C nachgerührt wurde, wird durch Destillation aufgearbeitet. Es werden 77,8 Teile 1-(3-Hydroxy-1-propyl)imidazol erhalten (Kp 154°/1 Torr) ; dies entspricht einer Ausbeute von 61,7 % der Theorie.

Beispiel 11

Herstellung von 1-(3,3-Dimethoxy-2-propyl)-imidazol

Unter den gleichen Bedingungen wie im Beispiel 10 beschrieben, wird die Mischung aus 119 Teilen 3,3-Dimethoxy-2-propylamin und 170 Teilen Ammoniakwasser 10 %ig mit einer Mischung aus 145 Teilen 40 %iger wäßriger Glyoxal-Lösung und 75 Teilen 40 %iger wäßriger Formaldehyd-Lösung umgesetzt. Durch Destillation des Reaktionsgemisches werden 133 Teile 1-(3,3-Dimethoxy-2-propyl)imidazol erhalten (Kp 124°/5 Torr), dies entspricht einer Ausbeute von 78,3 % der Theorie.

Beispiel 12

Herstellung von 1-(3-Methylamino-1-propyl)-imidazol

Eine Mischung aus 176 Teilen 3-Methylamino-1-propylamin, 200 Teilen Wasser und 107 Teilen Ammonchlorid sowie eine Mischung aus 290 Teilen 40 %iger wäßriger Glyoxal-Lösung und 150 Teilen 40 %iger Formaldehyd-Lösung werden gleichzeitig während 30 Minuten bei 70 °C in 100 Teile vorgelegtes Wasser eingerührt. Es wird 20 Minuten nachgerührt., anschließend mit 200 Teilen 40 %iger wäßriger Natronlauge neutralisiert und destillativ aufgearbeitet. Man erhält 115,6 Teile 1-(3-Methylamino-1-propyl)imidazol Kp 125°/2 Torr. Dies entspricht einer Ausbeute von 41,5 % der Theorie.

Beispiel 13

Herstellung von 1-(2-Äthoxy-1-äthyl)-imidazol

Unter den gleichen Bedingungen wie im Beispiel 10 beschrieben werden eine Mischung aus 89 Teilen 2-Äthoxy-1-äthylamin und 461 Teilen Ammoniakwasser 3,7 %ig sowie eine Mischung aus 145 Teilen 40 %iger wäßriger Glyoxal-Lösung und 75 Teilen 40 %iger wäßriger Formaldehyd-Lösung zur Umsetzung gebracht. Durch destillative Aufarbeitung werden 96,2 Teile 1-(2-Äthoxy-1-äthyl)imidazol erhalten (Kp 82°/1 Torr). Dies entspricht einer Ausbeute von 68,7 %.

**Ansprüche**

1. Verfahren zur Herstellung von in 1-Stellung substituierten Imidazolen, dadurch gekennzeichnet, daß man eine α-Dicarbonylverbindung, Ammoniak, einen Aldehyd und ein primäres Amin in wäßrigem Medium in einer Stufe bei Temperaturen von 20 bis 150 °C umsetzt.

2. Verfahren zur Herstellung von Imidazolen der Formel

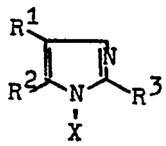

in der $R^1$, $R^2$ und $R^3$ Wasserstoffatome oder aliphatische oder aromatische Reste und X einen aliphatischen, cycloaliphatische oder aromatischen Reste bedeuten, dadurch gekennzeichnet, daß man eine α-Dicarbonylverbindung der Formel

$$R^1\text{—CO—CO—}R^2,$$

Ammoniak, einen Aldehyd der Formel $R^3$—CHO und ein primäres Amin der Formel X—$NH_2$ in einer Weise bei Temperaturen von 20 bis 150 °C umsetzt.

3. Verfahren nach Anspruch 1, dadurch gekennzeichnet, daß man als α-Dicarbonylverbindung Glyoxal, Diacetyl, Benzil oder Methylglyoxal verwendet.

4. Verfahren nach Anspruch 2, dadurch gekennzeichnet, daß man als Aldehyd Formaldehyd, Acetaldehyd, Propionaldehyd, Isobutyraldehyd oder Benzaldehyd verwendet.

5. Verfahren nach Anspruch 1, dadurch gekennzeichnet, daß man Amine der Formel X'-$NH_2$ verwendet, in der X' einen geradkettigen oder verzweigten, gesättigten oder ungesättigten Kohlenwasserstoffrest mit 1 bis 20 C-Atomen, der durch HO-, $CH_3$-NH-, $(CH_3)_2N$-, $H_5C_2NH$-, $H_5C_2O$-, $H_3CO$- oder Äthergruppen substituiert sein kann, einen cycloaliphatischen Rest oder einen Phenylrest, der durch $H_3CO$-, $(CH_3)_2N$-, $H_5C_2NH$- oder $H_3COOC$-Gruppe substituiert sein kann, bedeutet.

**Claims**

1. A process for the preparation of 1-substituted imidazoles, wherein an α-dicarbonyl compound, ammonia, an aldehyde and a primary amine are reacted in an aqueous medium, in a single stage, at 20-150 °C.

2. A process for the preparation of imidazoles of the formula

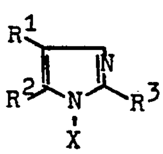

where $R^1$, $R^2$ and $R^3$ are hydrogens or aliphatic or aromatic radicals and X is an aliphatic, cycloaliphatic or aromatic radical, wherein an α-dicarbonyl compound of the formula

$$R^1—CO—CO—R^2,$$

ammonia, an aldehyde of the formula $R^3$—CHO and a primary amine of the formula X—$NH_2$ are reacted in one step at from 20 to 150 °C.

3. A process as claimed in claim 1, wherein the α-dicarbonyl compounds used is glyoxal, diacetyl, benzil or methylglyoxal.

4. A process as claimed in claim 2, wherein the aldehyde used is formaldehyde, acetaldehyde, propionaldehyde, isobutyraldehyde or benzaldehyde.

5. A process as claimed in claim 1, wherein the amine used is of the formula X'—$NH_2$, where X' is a straight-chain or branched, saturated or unsaturated hydrocarbon radical of 1 to 20 carbon atoms which may be substituted by HO-, $CH_3$-NH-, $(CH_3)_2N$-, $H_5C_2NH$-, $H_5C_2O$-, $H_3CO$- or ether groups, or is a cycloaliphatic radical or is a phenyl radical which may be substituted by $H_3CO$-, $(CH_3)_2N$-, $H_5C_2NH$- or $H_3COOC$-groups.

**Revendications**

1. Procédé de préparation d'imidazoles substitués en position 1, caractérisé en ce que l'on fait réagir un composé α-dicarbonylé, de l'ammoniac, un aldéhyde et une amine primaire en milieu aqueux, en un seul stade, à des températures de 20 à 150 °C.

2. Procédé de préparation d'imidazoles de la formule

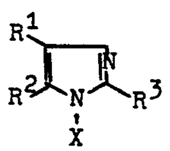

dans laquelle $R^1$, $R^2$ et $R^3$ représentent des atomes d'hydrogène ou des groupes aliphatiques ou aromatiques et X désigne un groupe aliphatique, cyclo-aliphatique ou aromatique, caractérisé en ce que l'on fait réagir un composé α-dicarbonylé de la formule

$$R^1—CO—CO—R^2,$$

de l'ammoniac, un aldéhyde de la formule $R^3$—CHO et une amine primaire de la formule X—$NH_2$ en un seul stade à des températures de 20 à 150 °C.

3. Procédé suivant la revendication 1, caractérisé en ce que le composé α-dicarbonylé est choisi parmi le glyoxal, le diacétyle, le benzile et le méthylglyoxal.

4. Procédé suivant la revendication 2, caractérisé en ce que l'aldéhyde est choisi parmi le formaldéhyde, l'acétaldéhyde, le propionaldéhyde, l'isobutyraldéhyde et le benzaldéhyde.

5. Procédé suivant la revendication 1, caractérisé en ce que l'on emploie des amines de la formule X'—$NH_2$, X' désignant un groupe hydrocarboné en $C_1$ à $C_{20}$, à chaîne droite ou ramifiée, saturé ou non, éventuellement substitué par des groupes HO-, $CH_3$-NH-, $(CH_3)_2N$-, $H_5C_2NH$-, $H_5C_2O$-, $H_3CO$- ou éther, un groupe cyclo-aliphatique ou un groupe phényle éventuellement substitué par des groupes $H_3CO$-, $(CH_3)_2N$-, $H_5C_2NH$- ou $H_3COOC$-.